# EUROPÄISCHE PATENTSCHRIFT

(11) **EP 3 379 542 B1**
(45) Veröffentlichungstag und Bekanntmachung des Hinweises auf die Patenterteilung: **31.01.2024**
(21) Anmeldenummer: 18000244.6
(22) Anmeldetag: 12.03.2018
(51) Int. Cl.: G16H 40/40, G16H 40/63, G16H 40/67, G16H 20/40, G08C 17/02

(54) **VERFAHREN UND VORRICHTUNG ZUR ÜBERMITTLUNG VON DATEN VON BEATMUNGSGERÄTEN**
METHOD AND DEVICE FOR TRANSMISSION OF VENTILATION DATA
PROCÉDÉ ET DISPOSITIF DE TRANSMISSION DE DONNÉES DE APPAREILS RESPIRATOIRES

(30) Priorität: 22.03.2017 DE 102017002750
(43) Veröffentlichungstag der Anmeldung: 26.09.2018
(73) Patentinhaber: Löwenstein Medical Technology S.A., 2557 Luxembourg (LU)
(72) Erfinder: Bychkov, Igor, 76275 Ettlingen (DE)
(74) Vertreter: Marx, Thomas

(56) Entgegenhaltungen:
- WO-A2-2004/070557
- US-A1- 2008 097 909
- US-A1- 2012 226 771
- GOVONI L ET AL: "An improved telemedicine system for remote titration and optimization of Home Mechanical Ventilation", BIOMEDICAL ENGINEERING CONFERENCE (CIBEC), 2010 5TH CAIRO INTERNATIONAL, IEEE, 16. Dezember 2010 (2010-12-16), Seiten 66-69, XP031979754, DOI: 10.1109/CIBEC.2010.5716060 ISBN: 978-1-4244-7168-3
- STEINFELD E F: "INTERNET-APPLIANCE TECHNOLOGY AUTOMATES TEST EQUIPMENT", EDN ELECTRICAL DESIGN NEWS.(TEXAS INSTRUMENT), REED BUSINESS INFORMATION, HIGHLANDS RANCH, CO, US, Bd. 45, Nr. 21, 12. Oktober 2000 (2000-10-12), XP001143050, ISSN: 0012-7515

## Beschreibung

Zum Datenaustausch sind Beatmungsgeräte oftmals mit einer Schnittstelle zum Datenaustausch mit einem Netzwerk ausgerüstet. Eine solche Schnittstelle kann kabelgebunden, beispielsweise als RJ45 Datenschnittstelle ausgeführt, oder kabellos, beispielsweise als WLAN, Mobilfunk-, IoT, M2M oder Bluetooth Schnittstelle ausgeführt sein.

Über eine solche Datenschnittstelle können eine Vielzahl von Daten ausgetauscht werden. Beispielsweise kann die Software des Beatmungsgerätes durch ein Update durch Zugriff auf entfernte Daten aktualisiert werden, wie in der DE 10 2015 008 946 A1 beschrieben.

Weiterhin können Behandlungsdaten, wie Einstellungen, Messwerte therapeutischer oder physiologischer Parameter oder auch Patientendaten an ein entferntes Gerät, beispielsweise an einen Server zur Abspeicherung und Verfügbarmachung für Dritte gesendet werden, wie in der EP 2 392 253 A1 beschrieben.

Die WO 2004070557 A2 betrifft ein System, das ein Medizingerät aufweist, welches eine Kommunikation mit einem zentral angeordneten Server initiiert. Während dieser Interaktion werden Status- und Programmierinformationen von dem Medizingerät an den Server gesendet. Der Server speichert die Informationen vorübergehend und sendet diese Information an das entsprechende Endgerät (PDA). Dort kann die Information angezeigt werden.

Die US 2012226771 A1 betrifft ein Fernüberwachungssystem zum Überwachen einer Vielzahl von medizinischen Geräten in einer Patienten- oder Pflegeeinrichtung. Das System umfasst einen Geräteintegrationsserver, der mit drahtlosen Relaismodulen in Kommunikation steht, um Datenpakete von den medizinischen Geräten zu empfangen, einschließlich einer Kennung und Daten für jedes medizinische Gerät. Das System enthält auch einen ausgehenden Webserver. Der Webserver ist so konfiguriert, dass er Webseiten mit den Daten der medizinischen Geräte zur Anzeige auf ersten und zweiten Fernüberwachungsgeräten bereitstellt, vorbehaltlich der Authentifizierung einer zugehörigen Datenanforderung von dem Überwachungsgerät.

Die US 2008097909 A1 offenbart generische Methoden für den Austausch von medizinischen Daten über drahtgebundene Verbindungen. Dabei offenbart die D3 die Übertragung von Daten von einem Medizingerät zur einer Relais-Station.

Die Publikation GOVONI L ET AL, "An improved telemedicine system for remote titration and optimization of Home Mechanical Ventilation", BIOMEDICAL ENGINEERING CONFERENCE (CIBEC), 2010 5TH CAIRO INTERNATIONAL, IEEE, (20101216), doi:10.1109/CIBEC.2010.5716060, ISBN 978-1-4244-7168-3, pages 66 - 69, XP031979754, offenbart ein Fernüberwachungs- und Fernsteuerungs-system zum Überwachen von Beatmungsgeräten.

Die Publikation STEINFELD E F, "INTERNET-APPLIANCE TECHNOLOGY AUTOMATES TEST EQUIPMENT", EDN ELECTRICAL DESIGN NEWS.(TEXAS INSTRUMENT), REED BUSINESS INFORMATION, HIGHLANDS RANCH, CO, US, (20001012), vol. 45, no. 21, ISSN 0012-7515, XP001143050, offenbart eine Methode um nicht netzwerkfähige Geräte, durch eine Erweiterung netzwerkfähig zu machen.

Prinzipiell besteht jedoch die Möglichkeit, dass ein unberechtigter Datenaustausch über die Schnittstelle stattfindet. Es ist die Aufgabe der vorliegenden Erfindung, ein Verfahren und eine Vorrichtung anzugeben, die eine Fernüberwachung der Beatmungs-Therapie ermöglichen, welche den Datenaustausch sicherer gestaltet, welche die übertragene Datenmenge reduziert und welche überhaupt erstmals eine Fernüberwachung der Beatmungs-Therapie ohne aufwändige medizinische oder diagnostische Daten ermöglicht.

Die Lösung dieser Aufgabe erfolgt erfindungsgemäß durch ein in den Ansprüchen definiertes Verfahren und/oder durch eine in den Ansprüchen definierte Vorrichtung.

Die Erfindung betrifft ein Verfahren zur Übermittlung von Daten von Beatmungsgeräten mit einer Vielzahl an Beatmungsgeräten mit jeweils wenigstens einer Schnittstelle zu einer Relais-Station und jeweils einem Datenkanal zu der Relais-Station, dadurch gekennzeichnet dass der Datenkanal für Daten des Beatmungsgerätes, die Nutzungsstunden und/oder die Therapiequalität repräsentieren, unidirektional vom Gerät zur Relais-Station ist, wobei die Nutzungsstunden als Nutzungsdauer zunächst in einem internen Datenspeicher des Beatmungsgerätes gespeichert und anschließend an die Relais-Station weitergeleitet wird und wobei die Nutzungsdauer des Beatmungsgerätes durch den Patienten unter Berücksichtigung der Anzahl von Tagen und/oder Nächten, an denen die Therapie mindestens für eine definierte Zeitdauer angewendet wurde, bestimmt wird, wobei der Datenkanal zumindest zwei zumindest teilweise redundante Technologien für den Datentransfer unterstützt, wobei die Daten des Beatmungsgerätes verschlüsselt werden und verschlüsselt zur Relais-Station übertragen werden und wobei die Relais-Station die Daten des Beatmungsgerätes entschlüsselt und wobei eine Authentifizierung von Beatmungsgerät und/oder Relais-Station erfolgt, anhand von hinterlegtem Code oder der Seriennummer des Beatmungsgerätes, indem die Relaisstation den hinterlegten Code oder die Seriennummer erkennt, wobei in der Relais-Station eine Speicherung der empfangenen Daten in einem Speicher mindestens für einen Zeitraum, der für eine Abholung oder Übermittlung der Daten an der Weiterleitungsschnittstelle ausreichend ist, erfolgt, wobei in der Relais-Station eine Zuordnung der Beatmungsgeräte und/oder ihrer Daten zu bestimmten Nutzern oder Nutzergruppen erfolgt, so dass die Daten spezifisch zur Weiterleitung bereitgestellt werden für den Nutzer oder Nutzergruppen, denen die Geräte und/oder ihre Daten zugeordnet sind, wobei die Relais-Station eine Weiterleitungs-Schnittstelle aufweist für die Vermittlung von Daten zu Gegenstellen, wobei die Daten von der Relais-Station verschlüsselt werden und dann über die Weiterleitungs-Schnittstelle an die Gegenstelle übermittelt werden, wobei die Gegenstelle die Daten entschlüsselt und wobei die Gegenstelle vor der Übermittlung authentifiziert wird und anhand der Authentifizierung Daten oder Nutzer oder Nutzergruppe selektiv übermittelt werden, wobei die Gegenstelle Code oder Seriennummer anhand von hinterlegten Daten oder Schlüsseln erkennt, wobei die Übermittlung von der Relais-Station/Push-Station oder von der Gegenstelle/ Pull-Stelle ausgelöst wird.

Die Erfindung betrifft auch eine Vorrichtung zur Übermittlung von Daten von Beatmungsgeräten mit einer Vielzahl an Beatmungsgeräten mit jeweils wenigstens einer Schnittstelle zu einer Relais-Station und jeweils einem Datenkanal zu der Relais-Station, dadurch gekennzeichnet dass der Datenkanal für Daten des Beatmungsgerätes, die Nutzungsstunden und/oder die Therapiequalität repräsentieren, unidirektional vom Gerät zur Relais-Station ist, wobei die Nutzungsstunden als Nutzungsdauer zunächst in einem internen Datenspeicher des Beatmungsgerätes gespeichert und anschließend an die Relais-Station weitergeleitet wird und wobei die Nutzungsdauer des Beatmungsgerätes durch den Patienten unter Berücksichtigung der Anzahl von Tagen und/oder Nächten, an denen die Therapie mindestens für eine definierte Zeitdauer angewendet wurde, bestimmt wird, wobei der Datenkanal zumindest zwei zumindest teilweise redundante Technologien für den Datentransfer unterstützt, wobei die Daten des Beatmungsgerätes verschlüsselt werden und verschlüsselt zur Relais-Station übertragen werden und wobei die Relais-Station die Daten des Beatmungsgerätes entschlüsselt und wobei eine Authentifizierung von Beatmungsgerät und/oder Relais-Station erfolgt, wobei in der Relais-Station eine Speicherung der empfangenen Daten in einem Speicher mindestens für einen Zeitraum, der für eine Abholung der Daten an der Weiterleitungsschnittstelle ausreichend ist erfolgt, wobei in der Relais-Station eine Zuordnung der Beatmungsgeräte und/oder ihrer Daten zu bestimmten Nutzern oder Nutzergruppen erfolgt, so dass die Daten spezifisch zur Weiterleitung bereitgestellt werden für den Nutzer oder Nutzergruppen, denen die Geräte und/oder ihre Daten zugeordnet sind, wobei die Relais-Station eine Weiterleitungs-Schnittstelle aufweist für die Vermittlung von Daten zu Gegenstellen, wobei die Daten von der Relais-Station verschlüsselt werden und dann über die Weiterleitungs-Schnittstelle an die Gegenstelle übermittelt werden, wobei die Gegenstelle die Daten entschlüsselt und wobei die Gegenstelle vor der Übermittlung authentifiziert wird und anhand der Authentifizierung Daten selektiv (für Nutzer / Nutzergruppe) übermittelt werden wobei die Übermittlung von der Relais-Station (Push) oder von der Gegenstelle (Pull, Abfrage) ausgelöst wird.

In einer möglichen Ausführungsform wird der Code zur Authentifizierung der Geräte, der Relais-Station, und / oder der Gegenstelle als Zertifikat ausgeführt, besonders bevorzugt mit einer zeitlich begrenzten Gültigkeit.

Die Erfindung ist auch dadurch gekennzeichnet, dass die zwei zumindest teilweise redundanten Technologien für den Datentransfer alternativ oder ergänzend sind 2G, 3G, 4G, 5G Mobilfunk mit Funkchips verschiedener Hersteller, WIFI + Internet, Bluetooth + Internet, Speicher-Karte + Internet, Lora, Siqfox oder andere Funkstandards für Maschine-zu-Maschine-Kommumkation.

Die Erfindung ist auch dadurch gekennzeichnet, dass die Relais-station ein realer oder virtueller Computer ist; zum Beispiel Webserver, Fileserver, ein Server im Krankenhaus oder in der Arztpraxis, ein mobiles Endgerät.

Die Erfindung ist auch dadurch gekennzeichnet, dass in der Relais-Station eine verschlüsselte Zwischenspeicherung der Daten erfolgt.

Die Erfindung ist auch dadurch gekennzeichnet, dass die Relais-station einen Benutzer-Zugang mit Konfigurations-Möglichkeiten zumindest für die Weiterleitungs-Schnittstelle, zum Beispiel Erzeugen von Tokens, aufweist.

Die Erfindung ist auch dadurch gekennzeichnet, dass über den Benutzer-Zugang die Zuordnung von Geräten zu Gegenstellen konfiguriert wird.

Die Erfindung ist auch dadurch gekennzeichnet, dass über den Benutzer-Zugang die Verarbeitung, Bearbeitung, Auswertung, Befundung, Archivierung oder Löschung der Daten aus den Geräten erfolgt.

Die Erfindung ist auch dadurch gekennzeichnet, dass über den Benutzer-Zugang die Fernkonfiguration der Geräte, zum Beispiel Wahl der Therapiedrücke und -modi, Verstellung der Befeuchtereinstellungen und Komforteinstellungen erfolgt.

Die Erfindung ist auch dadurch gekennzeichnet, dass über den Benutzer-Zugang ein Fernservice der Geräte, Auswerten von Fehlermeldungen, FW-Update aus der Ferne et cetera erfolgt.

Die Erfindung ist auch dadurch gekennzeichnet, dass der Benutzer-Zugang eine Authentifizierung der Benutzer, zum Beispiel über eine 2-Faktor Authentifizierung, durchführt.

Die Erfindung ist auch dadurch gekennzeichnet, dass die Relais-station die von den Geräten empfangenen Daten nach Abholung an der Weiterleitungs-Schnittstelle aus dem Speicher löscht.

Die Erfindung ist auch dadurch gekennzeichnet, dass die Relais-station die von den Geräten empfangenen Daten nach einem festen Zeitraum, der für Nutzer oder Nutzergruppe einstellbar ist, löscht.

Die Erfindung ist auch dadurch gekennzeichnet, dass die Relais-station die empfangenen Daten nach einem Lösch-Befehl, der zum Beispiel über die Weiterleitungs-Schnittstelle empfangen wird, löscht.

Die Erfindung ist auch dadurch gekennzeichnet, dass die Relais-station einen Datenkanal zum Patienten aufweist, zum Beispiel für Sprache, Video oder Benutzereingaben des Patienten an einem mobilen Endgerät zum Beispiel Fragebogen am Smartphone.

Die Erfindung ist auch dadurch gekennzeichnet, dass die Weiterleitungs-Schnittstelle auf Basis von HTTP oder HTTPS über Rechnernetze wie das Internet Daten austauscht und auf entfernten Computern Funktionen aufruft.

Die Erfindung ist auch dadurch gekennzeichnet, dass die Weiterleitungs-Schnittstelle einen Uniform Resource Identifier URI aufweist, über den sie eindeutig identifizierbar ist, sowie eine Schnittstellenbeschreibung in maschinenlesbarem Format als XML-Artefakt, zum Beispiel WSDL, die definiert, wie mit dem Weiterleitungs-Schnittstelle zu interagieren ist.

Die Erfindung ist auch dadurch gekennzeichnet, dass die Kommunikation mit der Weiterleitungs-Schnittstelle über Protokolle aus dem Internetkontext wie HTTP erfolgt und XML oder JSON basiert ist.

Die Erfindung ist auch dadurch gekennzeichnet, dass die Weiterleitungs-Schnittstelle eine REST-Architektur aufweist.

Die Erfindung ist auch dadurch gekennzeichnet, dass die Weiterleitungs-Schnittstelle eine Verschlüsselung mit https durchführt.

Die Erfindung ist auch dadurch gekennzeichnet, dass die Weiterleitungs-Schnittstelle die Relais-Station und die Gegenstelle unmittelbar miteinander verbindet, zum Beispiel über eine Web-API. Eine Zwischenablage, zum Beispiel in Form von Dateien die auf einem (S)FTP-Server abgelegt werden, ist nicht erforderlich. Dies reduziert die Gefahr des unbefugten Zugriffes durch Dritte und ermöglicht eine unmittelbare Erfolgskontrolle der Daten-Weiterleitung.

Die Erfindung ist auch dadurch gekennzeichnet, dass die Weiterleitungs-Schnittstelle die Gegenstelle anhand einer Nutzer-/Nutzergruppen-Kennung und von SW-Tokens authentifiziert. Die Erfindung ist auch dadurch gekennzeichnet, dass die Gegenstelle die Daten eines bestimmten Gerätes zum Beispiel identifiziert über die Seriennummer abfragen kann. Ist dieses dem Nutzer oder der Nutzergruppe, für den sich die Gegenstelle authentifiziert, in der Zuordnungstabelle der Relais-Station zugeordnet, werden die Daten übermittelt.

Die Erfindung ist auch dadurch gekennzeichnet, dass die Gegenstelle Daten ohne eine bestimmte Seriennummer anfragen, dann erhält der Nutzer oder die Nutzergruppe, für die sich die Gegenstelle authentifiziert, die Daten aller Geräte, die ihm / ihr in der Zuordnungstabelle der Relais-Station zugeordnet sind. Somit wird sichergestellt, dass die Daten aller Geräte, die ein Nutzer oder eine Nutzergruppe an der Relais-Station anmeldet, auch weitergeleitet werden. Eine Zuordnung zum Patienten erfolgt bevorzugt in der Gegenstelle.

Die Erfindung ist auch dadurch gekennzeichnet, dass für jeden Nutzer / Nutzergruppe in der Relais-Station eine Untermenge an Geräte-Daten festgelegt werden kann, die an der Weiterleitungs-Schnittstelle übermittelt werden soll, zum Beispiel nur Nutzungsdaten, Leckage, nur AHI. Diese Festlegung erfolgt bevorzugt durch mindestens einen Administrator der Relais-station oder alternativ durch verschiedene Abfrage-Kommandos von der Gegenstelle.

Die Erfindung ist auch dadurch gekennzeichnet, dass bei jeder Veränderung des Inhaltes des Speichers der Relais-Station, zum Beispiel bei jedem von einem Therapiegerät empfangenen Datensatz, ein Zähler erhöht wird. Fragt eine Gegenstelle Daten ab, so wird ihr auch der aktuelle Zählerstand über die Weiterleitungs-Schnittstelle übermittelt. Bei der nächsten Abfrage kann die Gegenstelle gezielt nach neuen Daten ab dem letzten ihr übermittelten Zählerstand abfragen. So wird die übermittelte Datenmenge reduziert auf die neu eingelaufenen Daten. Gleichzeitig bleibt die Relais-Station zustandslos, und kann an ihrer Weiterleitungs-Schnittelle von einer Vielzahl von Gegenstellen abgefragt werden, ohne für jede einzelne Gegenstelle speichern zu müssen, welchen Datensatz diese als letztes empfangen hat.

Die Erfindung ist auch dadurch gekennzeichnet, dass bei einer Abfrage neuer Daten durch die Gegenstelle die jeweilige Anzahl an übertragenen Datensätzen durch eine Obergrenze limitiert werden kann, um eine Überlastung einer Schnittstelle, eines Netzwerkes, eines Datenspeichers oder einer Datenverarbeitung zu vermeiden.

Die Erfindung ist auch dadurch gekennzeichnet, dass eine Gegenstelle mit einem einzigen Token die Daten vieler Nutzer / Nutzergruppen abfragen kann, sofern der Nutzer auf der Relais-station die Gegenstelle dazu ermächtigt hat, für ihn Daten abzuholen. Dazu übermittelt die Gegenstelle und die Relais-station während der Abfrage von Daten mindestens eine Kennung mindestens eines Nutzers. Die Relais-Station prüft, ob die Gegenstelle zur Abfrage von Daten dieses Nutzers berechtigt ist, und übermittelt im Fall des Vorliegens der Berechtigung die Daten von Geräten dieses Nutzers. Alternativ übermittelt die Relais-Station die Daten aller Geräte aller Nutzer, die die Gegenstelle zur Abfrage ihrer Daten berechtigt haben, und ergänzt als Attribut für jeden Datensatz eine Kennung mindestens eines Nutzers, dem das Gerät zugeordnet ist, damit die Gegenstelle nur diesem Nutzer den betreffenden Datensatz zur Verfügung stellt.

Die Erfindung betrifft auch ein Beatmungsgerät zur Verwendung in einer erfindungsgemäßen Vorrichtung / Verfahren.

Die Erfindung betrifft auch eine Relaisstation zur Verwendung in einer erfindungsgemäßen Vorrichtung / Verfahren.

Die Erfindung betrifft im Wesentlichen Beatmungsgeräte wie die APAP-Geräte, Bilevel-Geräte, Servoventilations-Geräte, Geräte für die Heimbeatmung und Intensivbeatmung sowie Notfallbeatmungsgeräte. Die Erfindung betrifft Beatmungsgeräte, die die Nutzungsdauer und die Therapiequalität ermitteln und speichern.

Die Therapiequalität kann beispielsweise unter Berücksichtigung der Anzahl zentraler, obstruktiver und gemischter Apnoen innerhalb einer bestimmten Zeiteinheit bestimmt werden.

Die Therapiequalität kann auch unter Berücksichtigung der Anzahl zentraler, obstruktiver und gemischter Hypopnoen oder RERAS oder Schnarchepochen oder Entsättigungen von SpO2 innerhalb einer bestimmten Zeiteinheit bestimmt werden.

Die Therapiequalität kann auch unter Berücksichtigung der Dauer und Stärke von Cheyne-Stokes-Atmung innerhalb einer bestimmten Zeiteinheit bestimmt werden.

Die Therapiequalität kann auch unter Berücksichtigung der Dauer und Stärke einer längerfristigen Hypoventilation, gemessen durch Atem(zeit)volumen-Reduktion und/oder Peakflow-Reduktion und/oder CO2-Anstieg und/oder Sauerstoff-Sättigungs-Abfall bestimmt werden.

Die Therapiequalität kann auch unter Berücksichtigung des Auftretens und der Dauer von nicht gewollten Situationen bestimmt werden, zum Beispiel das Auftreten hoher Leckagen oder Diskonnektionen, das Auftreten einer fehlenden Synchronität zwischen Beatmungsgerät und Patient oder das Auftreten von Alarmsituationen bzw. technischen Fehlern.

Die Therapiequalität wird zunächst in einem internen Datenspeicher des Gerätes gespeichert und anschließend an die Relais-Station weitergeleitet.

Die Nutzungsdauer des Beatmungsgerätes durch den Patienten kann beispielsweise unter Berücksichtigung der Anzahl von Tagen und/oder Nächten, an denen die Therapie mindestens für eine definierte Zeitdauer angewendet wurde, bestimmt werden.

Die Nutzungsdauer des Beatmungsgerätes kann auch als Zeitdauer für einen einzelnen Tag bestimmt werden, während der eine Therapie stattgefunden hat.

Die Nutzungsdauer des Beatmungsgerätes kann auch als Anteil von Tagen und/oder Nächten, an denen die Therapie mindestens für eine definierte Zeitdauer angewendet wurde, bestimmt werden.

Die Nutzungsdauer des Beatmungsgerätes kann auch als durchschnittliche Therapiedauer pro Kalender-Tag und/oder Nacht oder als Zeitraum innerhalb dessen das Beatmungsgerät eingeschaltet war bestimmt werden.

Dabei können bestimmte Bedingungen definiert werden, welche Zeitabschnitte bei eingeschaltetem für die Therapiedauer akzeptiert werden, zum Beispiel Maskenleckage unterhalb eines bestimmten Schwellwertes oder Detektion von Atemzügen oder Verwendung eines Atemluftbefeuchters.

Die Nutzungsdauer wird zunächst in einem internen Datenspeicher des Gerätes gespeichert und anschließend an die Relais-Station weitergeleitet.

Die Wartung/Funktion des Beatmungsgerätes kann beispielsweise unter Berücksichtigung der Einhaltung der Intervalle für die Reinigung und/oder den Austausch mindestens eines Luftfilters, Schlauches, Patienteninterfaces bestimmt werden.

Die Wartung/Funktion des Beatmungsgerätes kann beispielsweise unter Berücksichtigung von erkannten und gespeicherten Fehlerzuständen des Beatmungsgerätes bestimmt werden.

Die Wartung/Funktion des Beatmungsgerätes kann beispielsweise unter Berücksichtigung von Versionsständen mindestens einer Gerätesoftware oder mindestens einer Geräteelektronik bestimmt werden.

Die Wartung/Funktion des Beatmungsgerätes kann beispielsweise unter Berücksichtigung von einer Seriennummer, einer Hersteller-ID, einer UID, einer Netzwerk-Teilnehmernummer und einer Kennzeichnung des Gerätetyps bestimmt werden.

Die Wartung/Funktion des Beatmungsgerätes kann beispielsweise unter Berücksichtigung des aktuellen Therapiemodus und der aktuellen Therapieeinstellungen, zum Beispiel Druck- und Frequenzeinstellungen, der aktuellen Geräteeinstellungen, zum Beispiel Sprache, Datum, Uhrzeit oder der aktuellen Komforteinstellungen, zum Beispiel Befeuchterstufe, initiale Druckabsenkung oder Autostart, bestimmt werden.

Die Wartung/Funktion wird zunächst in einem internen Datenspeicher des Gerätes gespeichert und anschließend an die Relais-Station weitergeleitet.

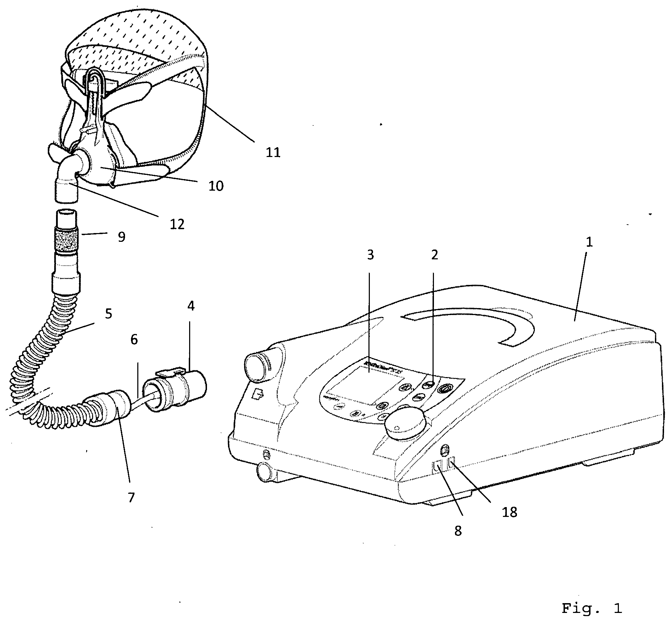

Fig. 1 zeigt den grundsätzlichen Aufbau eines Beatmungsgerätes (1). Im Bereich eines Gerätegehäuses mit Bedienfeld (2) sowie Anzeige (3) ist in einem Geräteinnenraum eine Atemgaspumpe angeordnet. Über eine Kopplung (4) wird ein Verbindungsschlauch (5) angeschlossen. Entlang des Verbindungsschlauches (5) kann ein zusätzlicher Druckmessschlauch (6) verlaufen, der über einen Druckeingangsstutzen (7) mit dem Gerätegehäuse verbindbar ist. Zur Ermöglichung einer Datenübertragung weist das Gerätegehäuse (1) zumindest eine Schnittstelle (8, 18) auf. Ein Anfeuchter kann adaptiert werden.

Im Bereich einer dem Gerätegehäuse (1) abgewandten Ausdehnung des Verbindungsschlauches (5) ist ein Ausatmungselement (9) angeordnet. Ebenfalls kann ein Ausatemventil verwendet werden.

Fig. 1 zeigt darüber hinaus ein als Beatmungsmaske (10) ausgebildetes Patienten-Interface, das hier beispielsweise als Nasalmaske realisiert ist. Eine Fixierung im Bereich eines Kopfes eines Patienten kann über eine Kopfhaube (11) erfolgen. Im Bereich seiner dem Verbindungsschlauch (5) zugewandten Ausdehnung weist das Patienten-Interface (10) ein Kupplungselement (12) auf.

Über die Schnittstelle (8, 18) kann die Eingabe und / oder Ausgabe von Daten, wie beispielsweise Totraumvolumen, erfolgen. Die Schnittstellen können kabelgebunden, als Infrarot-Schnittstelle, als Bluetooth-Schnittstelle, Mobilfunk-Schnittstelle, IoT bzw. M2M Schnittstelle oder USB realisiert sein.

Das erfindungsgemäße Beatmungsgerät (1) ist so ausgelegt, dass es über einen Schlauch und ein Patienteninterface mit einem Patienten verbunden werden kann, um eine Beatmung bereitzustellen. Es umfasst eine Quelle für Atemgas, die beispielsweise als ein Elektromotor mit Gebläserad ausgebildet ist und eine Einrichtung zur Ermittlung von Druck und/oder Fluss und/oder Volumen des Atemgases, sowie eine Steuereinheit, die so ausgelegt ist, dass sie für jeden Atemzyklus auf der Basis eines vorbestimmten Wertes für den Patienten und/oder auf Basis von Messsignalen für die Parameter Druck und/oder Flow und/oder Volumen einen Atemgasdruck bestimmt und die Quelle für Atemgas derart reguliert, dass der Atemgasdruck erzeugt wird.

Das Beatmungsgerät hat einen Speicher (31) zur Speicherung von Daten, die die Nutzungsdauer und die Therapiequalität und ergänzend die Funktion / Wartung des Gerätes repräsentieren.

Die Steuereinheit ist ferner so ausgelegt, dass sie den aktuellen Druck und/oder Flow und/oder das Volumen von Atemgas bestimmt und den aktuellen Wert über eine mit der Steuereinheit verbundene Anzeige darstellt. Die Steuereinheit ist außerdem so ausgelegt, dass sie bezogen auf einen oder mehrere Parameter Trendveränderungen ihrer Berechnungen über der Zeit bestimmt, wobei die Trendveränderungen auf der Anzeige angezeigt werden können.

Über ein Modem oder eine andere Schnittstelle (18) können ebenfalls aufgezeichnete Daten übermittelt werden.

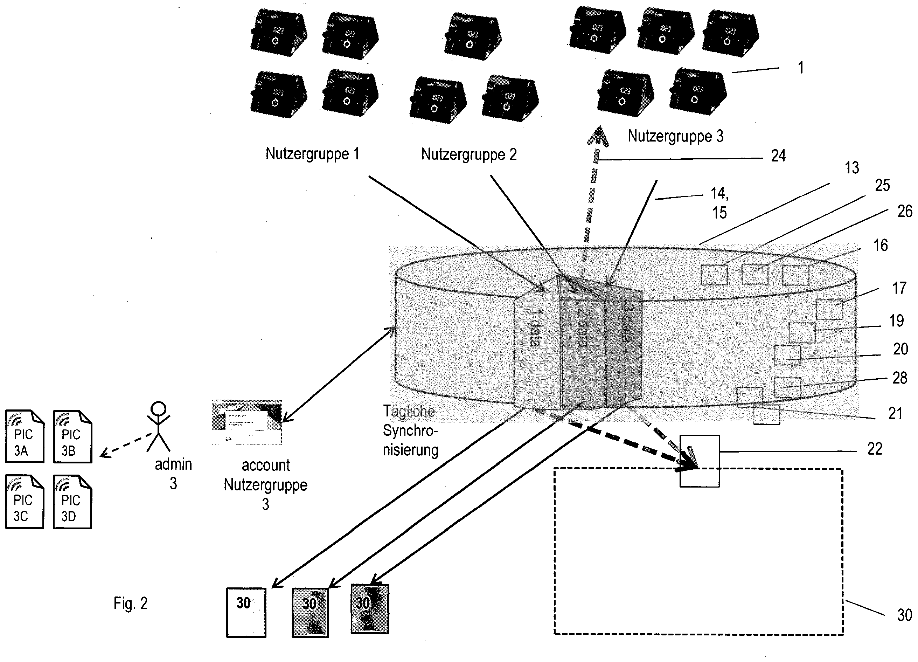

Figur 2 zeigt ein Vorrichtung mit einer Vielzahl an Beatmungsgeräten (1) mit jeweils wenigstens einer Schnittstelle (8, 18) zu einer Relais-Station (13) und jeweils einem Datenkanal (14) zu der Relais-Station, wobei der Datenkanal (14) zumindest für Daten des Beatmungsgerätes, die Nutzungsstunden oder die Therapiequalität repräsentieren, unidirektional vom Gerät (1) zur Relais-Station (13) ist. Der Datenkanal (14) unterstützt zumindest zwei zumindest teilweise redundante Technologien (15) für den Datentransfer, wobei eine Verschlüsselung (16) des Datenkanals erfolgt und wobei eine Authentifizierung (17) von Beatmungsgerät (1) und Relais-Station (13) erfolgt. In der Relais-Station (13) erfolgt eine Speicherung der empfangenen Daten in einem Speicher (19) für mindestens einen Zeitraum, der für eine Abholung der Daten an der Weiterleitungs-Schnittstelle (28) ausreichend ist. In der Relais-Station erfolgt zudem eine Zuordnung (20) der Beatmungsgeräte und/oder ihrer Daten zu bestimmten Nutzern oder Nutzergruppen, so dass die Daten spezifisch für den Nutzer oder Nutzergruppe, denen die Geräte und/oder ihre Daten zugeordnet sind, zur Weiterleitung bereitgestellt werden.

Die Relais-Station (13) weist eine Weiterleitungs-Schnittstelle (28) auf für die Vermittlung von Daten zu Gegenstellen (30) (zum Beispiel Webservern, PCs mit ERP-SW, et cetera), wobei die Daten von der Relais-Station (13) verschlüsselt (21) werden und dann über die Weiterleitungs-Schnittstelle (28) an die Gegenstelle (30) übermittelt werden. Die Gegenstelle (30) wird vor der Übermittlung authentifiziert (22) und anhand der Authentifizierung werden Daten (Nutzer oder Nutzergruppe) selektiv übermittelt, wobei die Übermittlung von der Relais-station (Push) oder von der Gegenstelle (Pull, Abfrage) ausgelöst werden kann.

Bei der Vorrichtung handelt es sich um ein drahtgebundenes oder drahtloses Telekommunikationsnetzwerk, wie zum Beispiel ein Computernetzwerk, ein Virtual Private Network (VPN), ein Intranet, Extranet oder das Internet.

Die Relaisstation (13) hat zumindest einen realen oder virtuellen Prozessor (26) zur Ausführung eines Programms (40) sowie eine Komponente zur Speicherung und/oder Generierung eines Datenschlüssels.

Zur Speicherung der Daten (32) vom Beatmungsgerät (1) wird beispielsweise wie folgt vorgegangen:
Mit Hilfe des Daten-Schlüssels (34) werden die Daten (32) verschlüsselt und von dem Beatmungsgerät über die Schnittstelle (18) und die Datenleitung (14) an die Relaisstation (13) übertragen. Die Relaisstation (13) speichert die verschlüsselten Daten in einem freien Speicherbereich.

Vorzugsweise erfolgt die Speicherung der verschlüsselten Daten zusammen mit einer Indexkennung des Beatmungsgerätes und /oder Nutzerdaten. Die Indexkennung des Beatmungsgerätes kann eine Seriennummer sein. Die Nutzerdaten können eine Patienten-ID oder Betreuer-ID sein.

Die Gegenstelle (30) kann die Daten eines bestimmten Gerätes (zum Beispiel identifiziert über die Seriennummer) abfragen. Ist dieses dem Nutzer oder der Nutzergruppe, für den sich die Gegenstelle (30) authentifiziert, in der Zuordnungstabelle (20) der Relais-Station (13) zugeordnet, werden die Daten übermittelt.

Alternativ kann die Gegenstelle (30) Daten ohne eine bestimmte Seriennummer anfragen, dann erhält der Nutzer oder die Nutzergruppe, für die sich die Gegenstelle (30) authentifiziert, die Daten aller Geräte, die ihm / ihr in der Zuordnungstabelle (20) der Relais-Station (13) zugeordnet sind. Somit wird sichergestellt, dass die Daten aller Geräte (1), die ein Nutzer oder eine Nutzergruppe an der Relais-Station (13) anmeldet, auch weitergeleitet werden. Eine Zuordnung zum Patienten erfolgt bevorzugt in der Gegenstelle (30).

Für jeden Nutzer / Nutzergruppe kann in der Relais-Station (13) eine Untermenge an Geräte-Daten festgelegt werden, die an der Weiterleitungs-Schnittstelle (28) übermittelt werden soll, zum Beispiel nur Leckage, nur AHI, ...Diese Festlegung erfolgt bevorzugt durch mindestens einen Administrator der Relais-station (13) oder alternativ durch verschiedene Abfrage-Kommandos von der Gegenstelle (30).

SyncID: Bei jeder Veränderung des Inhaltes des Speichers (19) der Relais-Station (13), zum Beispiel bei jedem von einem Therapiegerät empfangenen Datensatz, wird ein Zähler erhöht. Fragt eine Gegenstelle (30) Daten ab, so wird ihr auch der aktuelle Zählerstand über die Weiterleitungs-Schnittstelle (28) übermittelt. Bei der nächsten Abfrage kann die Gegenstelle (30) gezielt nach neuen Daten ab dem letzten ihr übermittelten Zählerstand abfragen. So wird die übermittelte Datenmenge reduziert auf die neu eingelaufenen Daten. Gleichzeitig bleibt die Relais-Station (13) zustandslos, und kann an ihrer Weiterleitungs-Schnittelle (28) von einer Vielzahl von Gegenstellen (30) abgefragt werden, ohne für jede einzelne Gegenstelle (30) speichern zu müssen, welchen Datensatz diese als letztes empfangen hat.

Eine Gegenstelle (30) kann (mit einem einzigen Token) die Daten vieler Nutzer / Nutzergruppen abfragen, sofern der Nutzer auf der Relais-Station (13) die Gegenstelle (30) dazu ermächtigt hat, für ihn Daten abzuholen. Dazu übermittelt die Gegenstelle (30) und die Relais-Station (13) während der Abfrage von Daten mindestens eine Kennung mindestens eines Nutzers. Die Relais-station (13) prüft, ob die Gegenstelle (30) zur Abfrage von Daten dieses Nutzers berechtigt ist, und übermittelt im Fall des Vorliegens der Berechtigung die Daten von Geräten dieses Nutzers. Alternativ übermittelt die Relais-Station die Daten aller Geräte aller Nutzer, die die Gegenstelle (30) zur Abfrage ihrer Daten berechtigt haben, und ergänzt als Attribut für jeden Datensatz eine Kennung mindestens eines Nutzers, dem das Gerät zugeordnet ist, damit die Gegenstelle (30) nur diesem Nutzer den betreffenden Datensatz zur Verfügung stellt.

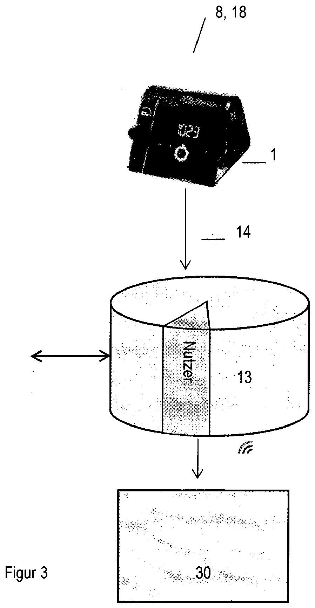

Figur 3 zeigt ein Vorrichtung mit einem Beatmungsgeräten (1) mit wenigstens einer Schnittstelle (8, 18) zu einer Relais-Station (13) und jeweils einem Datenkanal (14) zu der Relais-Station, wobei der Datenkanal (14) zumindest für Daten des Beatmungsgerätes, die Nutzungsstunden oder die Therapiequalität repräsentieren, unidirektional vom Gerät (1) zur Relais-Station (13) ist. Der Datenkanal (14) unterstützt zumindest zwei zumindest teilweise redundante Technologien (15) für den Datentransfer, wobei eine Verschlüsselung (16) des Datenkanals erfolgt und wobei eine Authentifizierung (17) von Beatmungsgerät (1) und Relais-Station (13) erfolgt. In der Relais-Station (13) erfolgt eine Speicherung der empfangenen Daten in einem Speicher (19) für mindestens einen Zeitraum, der für eine Abholung der Daten an der Weiterleitungs-Schnittstelle (28) ausreichend ist. In der Relais-Station erfolgt zudem eine Zuordnung (20) der Beatmungsgeräte und/oder ihrer Daten zu bestimmten Nutzern oder Nutzergruppen, so dass die Daten spezifisch für den Nutzer oder Nutzergruppe, denen die Geräte und/oder ihre Daten zugeordnet sind, zur Weiterleitung bereitgestellt werden.

Die Relais-Station (13) weist eine Weiterleitungs-Schnittstelle (28) auf für die Vermittlung von Daten zu Gegenstellen (30) (zum Beispiel Webservern, PCs mit ERP-SW, et cetera), wobei die Daten von der Relais-Station (13) verschlüsselt (21) werden und dann über die Weiterleitungs-Schnittstelle (28) an die Gegenstelle (30) übermittelt werden. Die Gegenstelle (30) wird vor der Übermittlung authentifiziert (22) und anhand der Authentifizierung werden Daten (Nutzer oder Nutzergruppe) selektiv übermittelt, wobei die Übermittlung von der Relais-Station (Push) oder von der Gegenstelle (Pull, Abfrage) ausgelöst werden kann.

Das Beatmungsgerät wird beispielsweise durch einlegen einer SD-Karte authentifiziert und einer Nutzergruppe zugewiesen. Die Authentifizierung erfolgt beispielsweise über ein Modem, welches mit dem Beatmungsgerät verbunden ist, an der Relais-Station (13). Die Relais-Station (13) leitet die Daten des Beatmungsgerätes bzw. der Authentifizierung an die Gegenstelle (30) weiter.

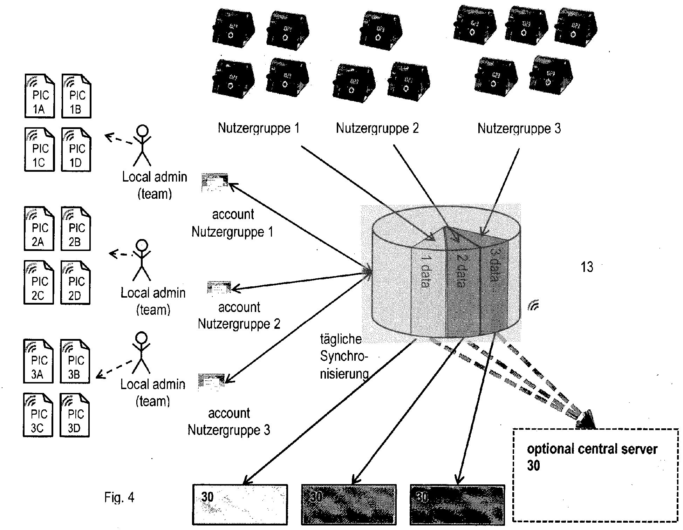

Figur 4 zeigt eine Vorrichtung mit vielen Beatmungsgeräten (1) die zu unterschiedlichen Nutzergruppen gebündelt sind. Die Beatmungsgeräte übermitteln Daten zu einer (oder mehreren) Relais-Station (13). Die Relaisstation (13) weist Nutzerspezifische (Speicher)-Bereiche auf. In der Relais-Station (13) erfolgt eine Speicherung der empfangenen Daten in einem Speicher (19) für mindestens einen Zeitraum, der für eine Abholung der Daten an der Weiterleitungs-Schnittstelle (28) ausreichend ist. In der Relais-Station erfolgt zudem eine Zuordnung (20) der Beatmungsgeräte und/oder ihrer Daten zu bestimmten Nutzern oder Nutzergruppen, so dass die Daten spezifisch für den Nutzer oder Nutzergruppe, denen die Geräte und/oder ihre Daten zugeordnet sind, zur Weiterleitung bereitgestellt werden.

Die Relais-Station (13) weist eine Weiterleitungs-Schnittstelle (28) auf für die Vermittlung von Daten zu Gegenstellen (30) (zum Beispiel Webservern, PCs mit ERP-SW, et cetera), wobei die Daten von der Relais-Station (13) verschlüsselt (21) werden und dann über die Weiterleitungs-Schnittstelle (28) an die Gegenstelle (30) übermittelt werden. Die Gegenstelle (30) wird vor der Übermittlung authentifiziert (22) und anhand der Authentifizierung werden Daten (Nutzer oder Nutzergruppe) selektiv übermittelt, wobei die Übermittlung von der Relais-station (Push) oder von der Gegenstelle (Pull, Abfrage) ausgelöst werden kann. Die Gegenstellen können physisch getrennte Gegenstellen sein. Es kann auch eine Gegenstelle (30) vorgesehen sein, die lediglich die Daten spezifisch für den Nutzer oder Nutzergruppe verwaltet.

Das Beatmungsgerät wird beispielsweise durch einlegen einer SD-Karte authentifiziert und einer Nutzergruppe zugewiesen. Die Authentifizierung erfolgt beispielsweise über ein Modem, welches mit dem Beatmungsgerät verbunden ist, an der Relais-Station (13). Die Relais-Station (13) leitet die Daten des Beatmungsgerätes bzw. der Authentifizierung an die Gegenstelle (30) weiter.

Administratoren der jeweiligen Nutzergruppe können über einen spezifischen Nutzergruppen-Account auf die Relaisstation zugreifen um beispielsweise Einstellungen der Relaisstation vorzunehmen oder Daten abzufragen.

## Patentansprüche

1. Verfahren zur Übermittlung von Daten von Beatmungsgeräten mit einer Vielzahl an Beatmungsgeräten mit jeweils wenigstens einer Schnittstelle zu einer Relais-Station und jeweils einem Datenkanal zu der Relais-Station, **dadurch gekennzeichnet dass** der Datenkanal für Daten des Beatmungsgerätes, die Nutzungsstunden und/oder die Therapiequalität repräsentieren, unidirektional vom Gerät zur Relais-Station ist, wobei die Nutzungsstunden als Nutzungsdauer zunächst in einem internen Datenspeicher des Beatmungsgerätes gespeichert und anschließend an die Relais-Station weitergeleitet wird und wobei die Nutzungsdauer des Beatmungsgerätes durch den Patienten unter Berücksichtigung der Anzahl von Tagen und/oder Nächten, an denen die Therapie mindestens für eine definierte Zeitdauer angewendet wurde, bestimmt wird, wobei der Datenkanal zumindest zwei zumindest teilweise redundante Technologien für den Datentransfer unterstützt, wobei die Daten des Beatmungsgerätes verschlüsselt werden und verschlüsselt zur Relais-Station übertragen werden und wobei die Relais-Station die Daten des Beatmungsgerätes entschlüsselt und wobei eine Authentifizierung von Beatmungsgerät und/oder Relais-Station erfolgt, anhand von hinterlegtem Code oder der Seriennummer des Beatmungsgerätes, indem die Relaisstation den hinterlegten Code oder die Seriennummer erkennt, wobei in der Relais-Station eine Speicherung der empfangenen Daten in einem Speicher mindestens für einen Zeitraum, der für eine Abholung oder Übermittlung der Daten an der Weiterleitungsschnittstelle ausreichend ist, erfolgt, wobei in der Relais-Station eine Zuordnung der Beatmungsgeräte und/oder ihrer Daten zu bestimmten Nutzern oder Nutzergruppen erfolgt, so dass die Daten spezifisch zur Weiterleitung bereitgestellt werden für den Nutzer oder Nutzergruppen, denen die Geräte und/oder ihre Daten zugeordnet sind, wobei die Relais-Station eine Weiterleitungs-Schnittstelle aufweist für die Vermittlung von Daten zu Gegenstellen, wobei die Daten von der Relais-Station verschlüsselt werden und dann über die Weiterleitungs-Schnittstelle an die Gegenstelle übermittelt werden, wobei die Gegenstelle die Daten entschlüsselt und wobei die Gegenstelle vor der Übermittlung authentifiziert wird und anhand der Authentifizierung Daten oder Nutzer oder Nutzergruppe selektiv übermittelt werden, wobei die Gegenstelle Code oder Seriennummer anhand von hinterlegten Daten oder Schlüsseln erkennt, wobei die Übermittlung von der Relais-Station/Push-Station oder von der Gegenstelle/ Pull-Stelle ausgelöst wird.

2. Vorrichtung zur Übermittlung von Daten von Beatmungsgeräten mit einer Vielzahl an Beatmungsgeräten (1) mit jeweils wenigstens einer Schnittstelle (8, 18) zu einer Relais-station (13) und jeweils einem Datenkanal (14) zu der Relais-station, **dadurch gekennzeichnet dass** der Datenkanal (14) für Daten des Beatmungsgerätes, die Nutzungsstunden und/oder die Therapiequalität repräsentieren, unidirektional vom Gerät (1) zur Relais-Station (13) ist, wobei die Nutzungsstunden als Nutzungsdauer zunächst in einem internen Datenspeicher des Beatmungsgerätes gespeichert und anschließend an die Relais-station weitergeleitet wird und wobei die Nutzungsdauer des Beatmungsgerätes durch den Patienten unter Berücksichtigung der Anzahl von Tagen und/oder Nächten, an denen die Therapie mindestens für eine definierte Zeitdauer angewendet wurde, bestimmt wird, wobei der Datenkanal (14) zumindest zwei zumindest teilweise redundante Technologien (15) für den Datentransfer unterstützt, wobei die Daten des Beatmungsgerätes verschlüsselt werden und verschlüsselt zur Relais-Station übertragen werden und wobei die Relais-Station (13) die Daten des Beatmungsgerätes entschlüsselt und wobei eine Authentifizierung (17) von Beatmungsgerät (1) und/oder Relais-Station (13) erfolgt, wobei in der Relais-Station (13) eine Speicherung der empfangenen Daten in einem Speicher (19) mindestens für einen Zeitraum, der für eine Abholung der Daten an der Weiterleitungsschnittstelle (28) ausreichend ist erfolgt, wobei in der Relais-Station eine Zuordnung (20) der Beatmungsgeräte und/oder ihrer Daten zu bestimmten Nutzern oder Nutzergruppen erfolgt, so dass die Daten spezifisch zur Weiterleitung bereitgestellt werden für den Nutzer oder Nutzergruppen, denen die Geräte und/oder ihre Daten zugeordnet sind, wobei die Relais-Station /13) eine Weiterleitungs-Schnittstelle (28) aufweist für die Vermittlung von Daten zu Gegenstellen (30), wobei die Daten von der Relais-Station (13) verschlüsselt (21) werden und dann über die Weiterleitungs-Schnittstelle (28) an die Gegenstelle (30) übermittelt werden, wobei die Gegenstelle (30) die Daten entschlüsselt und wobei die Gegenstelle (30) vor der Übermittlung authentifiziert (22) wird und anhand der Authentifizierung Daten selektiv (für Nutzer / Nutzergruppe) übermittelt werden wobei die Übermittlung von der Relais-station (Push) oder von der Gegenstelle (Pull, Abfrage) ausgelöst wird.

3. Vorrichtung nach zumindest einem der vorhergehenden Ansprüche **dadurch gekennzeichnet, dass** die Relais-Station (13) ein realer oder virtueller Computer ist und in der Relais-Station (13) eine verschlüsselte Zwischenspeicherung (19) der Daten erfolgt.

4. Vorrichtung nach zumindest einem der vorhergehenden Ansprüche **dadurch gekennzeichnet, dass** die Relais-Station (13) einen Benutzer-Zugang (25) mit Konfigurations-Möglichkeiten zumindest für die Weiterleitungs-Schnittstelle aufweist.

5. Vorrichtung nach zumindest einem der vorhergehenden Ansprüche **dadurch gekennzeichnet, dass** über den Benutzer-Zugang (25) die Zuordnung von Geräten (1) zu Gegenstellen (30) konfiguriert wird und/oder dass über den Benutzer-Zugang (25) die Verarbeitung, Bearbeitung, Auswertung, Befundung, Archivierung oder Löschung der Daten aus den Geräten (1) erfolgt.

6. Vorrichtung nach zumindest einem der vorhergehenden Ansprüche **dadurch gekennzeichnet, dass** über den Benutzer-Zugang (25) die Fernkonfiguration der Geräte (1), zum Beispiel Wahl der Therapiedrücke und -modi, Verstellung der Befeuchtereinstellungen und Komforteinstellungen erfolgt und dass über den Benutzer-Zugang (25) ein Fernservice der Geräte erfolgt und dass der Benutzer-Zugang (25) eine Authentifizierung der Benutzer durchführt.

7. Vorrichtung nach zumindest einem der vorhergehenden Ansprüche **dadurch gekennzeichnet, dass** die Relais-Station (13) die von den Geräten (1) empfangenen Daten nach Abholung an der Weiterleitungs-Schnittstelle (28) aus dem Speicher (19) löscht und/oder dass die Relais-Station (13) die von den Geräten (1) empfangenen Daten nach einem festen Zeitraum, der für Nutzer oder Nutzergruppe einstellbar ist, löscht und/oder dass die Relais-Station (13) die empfangenen Daten nach einem Lösch-Befehl, der über die Weiterleitungs-Schnittstelle (28) empfangen wird, löscht.

8. Vorrichtung nach zumindest einem der vorhergehenden Ansprüche **dadurch gekennzeichnet, dass** die Relais-Station (13) einen Datenkanal (24) zum Patienten aufweist.

9. Vorrichtung nach zumindest einem der vorhergehenden Ansprüche **dadurch gekennzeichnet, dass** die Weiterleitungs-Schnittstelle (28) auf Basis von HTTP oder HTTPS über Rechnernetze wie das Internet Daten austauscht und auf entfernten Computern (30) Funktionen aufruft und/oder dass die Kommunikation mit der Weiterleitungs-Schnittstelle (28) über Protokolle aus dem Internetkontext wie HTTP erfolgt und XML oder JSON basiert ist.

10. Vorrichtung nach zumindest einem der vorhergehenden Ansprüche **dadurch gekennzeichnet, dass** die Weiterleitungs-Schnittstelle (28) einen Uniform Resource Identifier (URI) aufweist, über den sie eindeutig identifizierbar ist, sowie eine Schnittstellenbeschreibung in maschinenlesbarem Format (als XML-Artefakt, zum Beispiel WSDL), die definiert, wie mit dem Weiterleitungs-Schnittstelle (28) zu interagieren ist.

11. Vorrichtung nach zumindest einem der vorhergehenden Ansprüche **dadurch gekennzeichnet, dass** die Weiterleitungs-Schnittstelle (28) eine REST-Architektur aufweist und/oder eine Verschlüsselung mit https durchführt und/oder die Weiterleitungs-Schnittstelle (28) die Gegenstelle (30) anhand einer Nutzer-/Nutzergruppen-Kennung und von SW-Tokens authentifiziert.

12. Vorrichtung nach zumindest einem der vorhergehenden Ansprüche **dadurch gekennzeichnet, dass** die Gegenstelle (30) die Daten eines bestimmten Gerätes abfragt und wenn dieses dem Nutzer oder der Nutzergruppe, für den sich die Gegenstelle (30) authentifiziert, in der Zuordnungstabelle (20) der Relais-Station (13) zugeordnet ist, die Daten übermittelt werden.

13. Vorrichtung nach zumindest einem der vorhergehenden Ansprüche **dadurch gekennzeichnet, dass** die Gegenstelle (30) Daten ohne eine bestimmte Seriennummer anfragt und der Nutzer oder die Nutzergruppe, für die sich die Gegenstelle (30) authentifiziert, die Daten aller Geräte erhält, die ihm / ihr in der Zuordnungstabelle (20) der Relais-Station (13) zugeordnet sind.

14. Vorrichtung nach zumindest einem der vorhergehenden Ansprüche **dadurch gekennzeichnet, dass** für jeden Nutzer / Nutzergruppe in der Relais-Station (13) eine Untermenge an Geräte-Daten festgelegt ist, die an der Weiterleitungs-Schnittstelle (28) übermittelt wird.

15. Vorrichtung nach zumindest einem der vorhergehenden Ansprüche **dadurch gekennzeichnet, dass** die Gegenstelle (30) die Daten vieler Nutzer / Nutzergruppen abfragt, sofern der Nutzer auf der Relais-Station (13) die Gegenstelle (30) dazu ermächtigt hat, für ihn Daten abzuholen, wozu die Gegenstelle (30) und die Relais-Station (13) während der Abfrage von Daten mindestens eine Kennung mindestens eines Nutzers übermittelt.

16. Vorrichtung nach zumindest einem der vorhergehenden Ansprüche **dadurch gekennzeichnet, dass** bei jeder Veränderung des Inhaltes des Speichers (19) der Relais-Station (13), ein Zähler verändert wird, wobei der Gegenstelle (30) bei einer Datenabfrage auch der aktuelle Zählerstand über die Weiterleitungs-Schnittstelle (28) übermittelt wird.

17. Beatmungsgerät (1) zur Verwendung in einer Vorrichtung nach Anspruch 2.

## Claims

1. A method for transmitting data from respirators, with a plurality of respirators each comprising at least one interface to a relay station and each comprising a data channel to the relay station, **characterized in that** the data channel for data of the respirator that represent hours of use and/or the therapeutic quality is unidirectional from the respirator to the relay station, wherein the hours of use are stored as the duration of use initially in an internal data memory of the respirator and subsequently forwarded to the relay station and wherein the duration of use of the respirator is determined by the patient in consideration of the number of days and/or nights on which the therapy is applied at least for a defined period of time, wherein the data channel supports at least two at least partially redundant technologies for the data transfer, wherein the data of the respirator are encrypted and are transmitted in encrypted form to the relay station and wherein the relay station decrypts the data of the respirator and wherein authentication of the respirator and/or relay station takes place, on the basis of the stored code or the serial number of the respirator **in that** the relay station recognizes the stored code or the serial number, wherein storage of the received data in a memory at least for a period of time which is sufficient for picking up or transmitting the data at the forwarding interface takes place in the relay station, wherein assignment of the respirators and/or their data to particular users or user groups takes place in the relay station, so that the data are specifically made available for forwarding for the user or user groups that are assigned the respirators and/or their data, wherein the relay station comprises a forwarding interface for transferring data to remote stations, wherein the data are encrypted by the relay station and then transmitted via the forwarding interface to the remote station, wherein the remote station decrypts the data and wherein the remote station is authenticated before the transmission and data or user or user group are selectively transmitted on the basis of the authentication, wherein the remote station recognizes the code or serial number on the basis of stored data or keys, wherein the transmission is initiated by the relay station/push station or by the remote station/pull station.

2. A device for transmitting data from respirators, with a plurality of respirators (1) each comprising at least one interface (8, 18) to a relay station (13) and each comprising a data channel (14) to the relay station, **characterized in that** the data channel (14) for data of the respirator that represent hours of use and/or the therapeutic quality is unidirectional from the respirator (1) to the relay station (13), wherein the hours of use is stored as the duration of use initially in an internal data memory of the respirator and subsequently forwarded to the relay station and wherein the duration of use of the respirator is determined by the patient in consideration of the number of days and/or nights on which the therapy is applied at least for a defined period of time, wherein the data channel (14) supports at least two at least partially redundant technologies (15) for the data transfer, wherein the data of the respirator are encrypted and are transmitted in encrypted form to the relay station and wherein the relay station (13) decrypts the data of the respirator and wherein authentication (17) of the respirator (1) and/or relay station (13) takes place, wherein storage of the received data in a memory (19) at least for a period of time which is sufficient for picking up the data at the forwarding interface (28) takes place in the relay station (13), wherein assignment (20) of the respirators and/or their data to particular users or user groups takes place in the relay station, so that the data are specifically made available for forwarding for the user or user groups that are assigned the respirators and/or their data, wherein the relay station (13) comprises a forwarding interface (28) for transferring data to remote stations (30), wherein the data are encrypted (21) by the relay station (13) and then transmitted via the forwarding interface (28) to the remote station (30), wherein the remote station (30) decrypts the data and wherein the remote station (30) is authenticated (22) before the transmission and data are selectively transmitted (for the user/user group) on the basis of the authentication, wherein the transmission is initiated by the relay station (push) or by the remote station (pull, query).

3. The device according to at least one of the preceding claims, **characterized in that** the relay station (13) is a real or virtual computer and encrypted intermediate storage takes place in the relay station (13).

4. The device according to at least one of the preceding claims, **characterized in that** the relay station (13) comprises a user access (25) with configuration possibilities at least for the forwarding interface.

5. The device according to at least one of the preceding claims, **characterized in that** the assignment of respirators (1) to remote stations (30) is configured via the user access (25) and/or in that the processing, editing, evaluation, diagnosis, archiving or deletion of the data from the respirators (1) takes place via the user access (25).

6. The device according to at least one of the preceding claims, **characterized in that** the remote configuration of the respirators (1), for example selection of the therapeutic pressures and modes, adjustment of the humidifier settings and comfort settings, takes place via the user access (25) and **in that** remote servicing of the respirators takes place via the user access (25) and **in that** the user access (25) carries out authentication of the users.

7. The device according to at least one of the preceding claims, **characterized in that** the relay station (13) deletes the data received from the respirators (1) from the memory (19) after said data have been picked up at the forwarding interface (28) and/or **in that** the relay station (13) deletes the data received from the respirators (1) after a fixed period of time that can be set for a user or user group, and/or **in that** the relay station (13) deletes the received data after a delete command received via the forwarding interface (28).

8. The device according to at least one of the preceding claims, **characterized in that** the relay station (13) comprises a data channel (24) for the patient.

9. The device according to at least one of the preceding claims, **characterized in that** the forwarding interface (28) exchanges data on the basis of HTTP or HTTPS over computer networks such as the Internet and calls up functions on remote computers (30) and/or **in that** the communication with the forwarding interface (28) takes place using protocols from an Internet context, such as HTTP, and is based on XML or JSON.

10. The device according to at least one of the preceding claims, **characterized in that** the forwarding interface (28) comprises a uniform resource identifier (URI) through which it can be uniquely identified, as well as an interface description in machine-readable format (as an XML artifact, for example W SDL), which defines how to interact with the forwarding interface (28).

11. The device according to at least one of the preceding claims, **characterized in that** the forwarding interface (28) comprises a REST architecture and/or carries out encryption with https and/or the forwarding interface (28) authenticates the remote station (30) by a user/user group identifier and with SW tokens.

12. The device according to at least one of the preceding claims, **characterized in that** the remote station (30) queries the data of a specific respirator and the data are transmitted if the respirator is assigned in the assignment table (20) of the relay station (13) to the user or user group for which the remote station (30) is authenticated.

13. The device according to at least one of the preceding claims, **characterized in that** the remote station (30) requests data without a specific serial number and the user or user group for which the remote station (30) is authenticated receives the data of all respirators that are assigned to it in the assignment table (20) of the relay station (13).

14. The device according to at least one of the preceding claims, **characterized in that** a subset of respirator data which is transmitted to the forwarding interface (28) is specified for each user/user group in the relay station (13).

15. The device according to at least one of the preceding claims, **characterized in that** the remote station (30) queries the data of many users/user groups, provided that the user has entitled the remote station (30) at the relay station (13) to pick up data for it, for which purpose the remote station (30) and the relay station (13) transmit at least one identifier of at least one user during the data query.

16. The device according to at least one of the preceding claims, **characterized in that** a counter is changed with each change to the content of the memory (19) of the relay station (13), wherein the current counter state is also transmitted to the remote station (30) via the forwarding interface (28) in the event of a data query.

17. A respirator (1) for use in a device according to claim 2.

## Revendications

1. Procédé de transmission de données d'appareils respiratoires avec une pluralité d'appareils respiratoires dotés respectivement d'au moins une interface vers une station-relais et respectivement d'un canal de données vers la station-relais, **caractérisé en ce que** le canal de données destiné à des données de l'appareil respiratoire représentant des heures d'utilisation et/ou la qualité thérapeutique est unidirectionnel entre l'appareil et la station-relais, dans lequel les heures d'utilisation sont tout d'abord enregistrées en tant que durée d'utilisation dans une mémoire de données interne de l'appareil respiratoire et ensuite routées vers la station-relais et dans lequel la durée d'utilisation de l'appareil respiratoire est déterminée par le patient en tenant compte du nombre de jours et/ou de nuits, pendant lesquels la thérapie a été appliquée au moins pour une durée définie, dans lequel le canal de données supporte au moins deux technologies au moins partiellement redondantes pour le transfert de données, dans lequel les données de l'appareil respiratoire sont chiffrées et transmises de manière chiffrée à la station-relais et dans lequel la station-relais déchiffre les données de l'appareil respiratoire et dans lequel une authentification de l'appareil respiratoire et/ou de la station-relais est effectuée à l'aide d'un code enregistré ou du numéro de série de l'appareil respiratoire, du fait que la station-relais reconnaît le code enregistré ou le numéro de série, dans lequel un enregistrement des données reçues dans une mémoire est effectué dans la station-relais au moins pour un laps de temps suffisant pour une récupération ou une transmission des données au niveau de l'interface de routage, dans lequel une attribution des appareils respiratoires et/ou de leurs données à des utilisateurs ou des groupes d'utilisateurs déterminés est effectuée dans la station-relais, de sorte que les données sont mises à disposition spécifiquement pour le routage pour l'utilisateur ou des groupes d'utilisateurs auxquels les appareils et/ou leurs données sont attribués, dans lequel la station-relais présente une interface de routage pour transmission de données à des stations distantes, dans lequel les données sont chiffrées par la station-relais et ensuite transmises à la station distante par le biais de l'interface de routage, dans lequel la station distante déchiffre les données et dans lequel la station distante est authentifiée avant la transmission et des données ou des utilisateurs ou des groupes d'utilisateurs sont transmis sélectivement à l'aide de l'authentification, dans lequel la station distante reconnaît des codes ou des numéros de série à l'aide de données enregistrées ou de clés, dans lequel la transmission est déclenchée par la station-relais/station-push ou par la station distante/le point pull.

2. Dispositif de transmission de données d'appareils respiratoires avec une pluralité d'appareils respiratoires (1) dotés respectivement d'au moins une interface (8, 18) vers une station-relais (13) et respectivement d'un canal de données (14) vers la station-relais, **caractérisé en ce que** le canal de données (14) destiné à des données de l'appareil respiratoire représentant des heures d'utilisation et/ou la qualité thérapeutique est unidirectionnel entre l'appareil (1) et la station-relais (13), dans lequel les heures d'utilisation sont tout d'abord enregistrées en tant que durée d'utilisation dans une mémoire de données interne de l'appareil respiratoire et ensuite routées vers la station-relais et dans lequel la durée d'utilisation de l'appareil respiratoire est déterminée par le patient en tenant compte du nombre de jours et/ou de nuits, pendant lesquels la thérapie a été appliquée au moins pour une durée définie, dans lequel le canal de données (14) supporte au moins deux technologies (15) au moins partiellement redondantes pour le transfert de données, dans lequel les données de l'appareil respiratoire sont chiffrées et transmises de manière chiffrée à la station-relais et dans lequel la station-relais (13) déchiffre les données de l'appareil respiratoire et dans lequel une authentification (17) de l'appareil respiratoire (1) et/ou de la station-relais (13) est effectuée, dans lequel un enregistrement des données reçues dans une mémoire (19) est effectué dans la station-relais (13) au moins pour un laps de temps suffisant pour une récupération des données au niveau de l'interface de routage (28), dans lequel une attribution (20) des appareils respiratoires et/ou de leurs données à des utilisateurs ou des groupes d'utilisateurs déterminés est effectuée dans la station-relais, de sorte que les données sont mises à disposition spécifiquement pour le routage pour l'utilisateur ou des groupes d'utilisateurs auxquels les appareils et/ou leurs données sont attribués, dans lequel la station-relais (13) présente une interface de routage (28) pour transmission de données à des stations distantes (30), dans lequel les données sont chiffrées (21) par la station-relais (13) et ensuite transmises à la station distante (30) par le biais de l'interface de routage (28), dans lequel la station distante (30) déchiffre les données et dans lequel la station distante (30) est authentifiée (22) avant la transmission et des données sont transmises sélectivement (pour un utilisateur / groupe d'utilisateurs) à l'aide de l'authentification, dans lequel la transmission est déclenchée par la station-relais (push) ou par la station distante (pull, consultation).

3. Dispositif selon l'une au moins des revendications précédentes, **caractérisé en ce que** la station-relais (13) est un ordinateur réel ou virtuel et **en ce qu'**un enregistrement intermédiaire chiffré est effectué dans la station-relais (13).

4. Dispositif selon l'une des revendications précédentes, **caractérisé en ce que** la station-relais (13) présente un accès utilisateur (25) avec des options de configuration au moins pour l'interface de routage.

5. Dispositif selon l'une au moins des revendications précédentes, **caractérisé en ce que** l'attribution d'appareils (1) à des stations distantes (30) est configurée par le biais de l'accès utilisateur (25) et/ou en ce que le traitement, l'édition, l'évaluation, l'interprétation, l'archivage ou la suppression de données provenant des appareils (1) sont effectués par le biais de l'accès utilisateur (25).

6. Dispositif selon l'une au moins des revendications précédentes, **caractérisé en ce que** la configuration à distance des appareils (1), par exemple le choix des pression et modes thérapeutiques, la modification des réglages de l'humidificateur et des réglages de confort, est effectuée par le biais de l'accès utilisateur (25) et **en ce qu'**une maintenance à distance des appareils est effectués par le biais de l'accès utilisateur (25) et **en ce qu'**une authentification des utilisateurs est effectués par le biais de l'accès utilisateur (25).

7. Dispositif selon l'une au moins des revendications précédentes, **caractérisé en ce que** la station-relais (13) supprime les données reçues depuis les appareils (1) de la mémoire (19) après récupération au niveau de l'interface de routage (28) et/ou **en ce que** la station-relais (13) supprime les données reçues depuis les appareils (1) après un laps de temps fixe, lequel peut être réglé pour des utilisateurs ou des groupes d'utilisateurs, et/ou **en ce que** la station-relais (13) supprime les données reçues après un ordre de suppression reçu par le biais de l'interface de routage (28).

8. Dispositif selon l'une au moins des revendications précédentes, **caractérisé en ce que** la station-relais (13) présente un canal de données (24) vers le patient.

9. Dispositif selon l'une au moins des revendications précédentes, **caractérisé en ce que** l'interface de routage (28) échange des données sur la base HTTP ou HTTPS par le biais de réseaux informatiques tels que l'Internet et appelle des fonctions sur des ordinateurs distants (30) et/ou **en ce que** la communication avec l'interface de routage (28) est effectuée par le biais de protocoles du contexte Internet tels que HTTP et se base sur XML ou JSON.

10. Dispositif selon l'une au moins des revendications précédentes, **caractérisé en ce que** l'interface de routage (28) présente un Uniform Resource Identifier (URI), par le biais duquel elle peut être identifiée de manière univoque, ainsi qu'une description d'interface au format lisible par machine (tel que XML-Artefact, par exemple WSDL), laquelle définit comment interagir avec l'interface de routage (28).

11. Dispositif selon l'une au moins des revendications précédentes, **caractérisé en ce que** l'interface de routage (28) présente une architecture REST et/ou exécute un chiffrage avec https et/ou l'interface de routage (28) authentifie la station distante (30) à l'aide d'un identifiant d'utilisateur /de groupes d'utilisateurs et de jetons SW.

12. Dispositif selon l'une au moins des revendications précédentes, **caractérisé en ce que** la station distante (30) consulte les données d'un appareil déterminé et les données sont transmises lorsque celui-ci est attribué à l'utilisateur ou au groupe d'utilisateurs pour lequel la station distante (30) est authentifiée, dans le tableau de correspondances (20) de la station-relais (13).

13. Dispositif selon l'une au moins des revendications précédentes, **caractérisé en ce que** la station distante (30) demande des données sans numéro de série déterminé et l'utilisateur ou le groupe d'utilisateurs pour lequel la station distante (30) est authentifiée obtient les données de tous les appareils qui lui sont attribués dans le tableau de correspondances (20) de la station-relais (13).

14. Dispositif selon l'une au moins des revendications précédentes, **caractérisé en ce que** pour chaque utilisateur / groupe d'utilisateurs, un sous-ensemble de données d'appareil est défini dans la station-relais (13), lequel est transmis au niveau de l'interface de routage (28).

15. Dispositif selon l'une au moins des revendications précédentes, **caractérisé en ce que** la station distante (30) consulte les données d'un grand nombre d'utilisateurs / de groupes d'utilisateurs, à condition que l'utilisateur ait autorisé la station distante (30) sur la station-relais (13) à récupérer des données pour lui, ce pour quoi la station distante (30) et la station-relais (13) transmettent au moins un identifiant d'au moins un utilisateur pendant la consultation de données.

16. Dispositif selon l'une au moins des revendications précédentes, **caractérisé en ce qu'**à chaque modification du contenu de la mémoire (19) de la station-relais (13), un compteur est modifié, dans lequel le relevé de compteur actuel est également transmis à la station distante (30) par le biais de l'interface de routage (28) lors d'une consultation de données.

17. Appareil respiratoire (1) destiné à être utilisé dans un dispositif selon la revendication 2.
